# EUROPEAN PATENT SPECIFICATION

(11) **EP 0 948 544 B1**
(45) Date of publication and mention of the grant of the patent: **02.05.2003**
(21) Application number: 97947810.4
(22) Date of filing: 10.12.1997
(51) Int. Cl.: C07K 16/00

(54) **MONOVALENT ANTIBODY FRAGMENTS**
MONOVALENTE ANTIKÖRPERFRAGMENTE
FRAGMENTS D'ANTICORPS MONOVALENTS

(30) Priority: 10.12.1996 GB 9625640
(43) Date of publication of application: 13.10.1999
(60) Divisional application: 01203352.8
(73) Proprietor: Celltech R&D Limited, Slough, Berkshire SL1 4EN (GB)
(72) Inventor: KING, David, John, Camberley Surrey GU15 2PF (GB); CHAPMAN, Andrew, Paul, Hampton Middlesex TW12 3DL (GB)
(74) Representative: Mercer, Christopher Paul
(86) International application number: GB9703400
(87) International publication number: WO98025971

(56) References cited:
- WO-A-96/09325
- PEDLEY R. B. ET AL.,: "The potential for enhanced tumour localisation by poly(ethylene glycol) modification of anti-CEA antibody" BR. J. CANCER, vol. 70, - 1994 pages 1126-1130, XP002061757
- CHIEN-TSUN KUAN ET AL: "PSEUDOMONAS EXTOXIN A MUTANTS REPLACEMENT OF SURFACE EXPOSED RESIDUES IN DOMAIN IN WITH CYSTEINERESIDUES THAT CAN BE MODIFIED WITH POLYETHYLENE GLYCOL IN A SITE-SPECIFIC MANNER" JOURNAL OF BIOLOGICAL CHEMISTRY, vol. 269, no. 10, 11 March 1994, pages 7610-7616, XP000563744
- WOGHIREN C. ET AL.,: "Protected Thiol-Polyethylen glycol: a new activated polymer for reversible protein modification" BIOCONJUGATE CHEM., vol. 4, - 1993 pages 314-318, XP002061758

## Description

This invention relates to modified monovalent antibody fragments, to processes for their preparation, to compositions containing them and to their use in medicine.

Antibodies are increasingly being used in the clinic for diagnostic and therapeutic purposes. The aim in each case is to exploit the combination of high specificity and affinity of the antibody-antigen interaction, to enable detection and/or treatment of a particular lesion. The antibody is used alone, or is loaded with another atom or molecule such as a radioisotope or cytotoxic drug.

The pharmacokinetics and biodistribution of an antibody play a major role in determining whether its use in the clinic will be successful. Thus the antibody must be capable of being delivered to the site of action and be retained there for a length of time suitable to achieve its purpose. It also should be present only at sub-toxic levels outside of the target and it must be catabolised in a well-defined manner.

For many uses the pharmacokinetics of antibodies are not ideal. This is especially true for tumour diagnosis and therapy with antibody-radioisotope or drug conjugates. For diagnosis with such conjugates long half-lives limit the tumour-to-background ratio and hence the sensitivity of lesion detection. For therapy, a long half-life leads to long-term exposure of normal tissues to the antibody conjugate and hence to dose-limiting toxicity.

A number of approaches are available to manipulate the pharmacokinetics of antibodies, and these usually also affect their biodistribution. The simplest and most generally applicable approach is the use of antibody fragments. These are cleared more rapidly from the circulation than whole antibodies and distribute more rapidly from the blood to the tissues, which is a particular advantage in some applications, for example for tumour imaging and therapy.

In order to improve the pharmocokinetics of antibody fragments still further we have investigated the use of polymers. The attachment of polymeric materials such as polyethylene glycol (PEG), to protein molecules is well established and it has been demonstrated that attachment of a polymer can substantially alter the pharmacological properties of a protein molecule. For example, PEG modification of proteins can alter the *in vivo* circulating half-life of the protein, antigenicity and immunogenicity, solubility, mechanical stability and resistance to proteolysis [Abuchowski, A. *et al* J. Biol. Chem (1977) 252, 3578-3581 and 3582-3586; Nucci, M. L. *et al*, Adv. Drug Delivery Reviews (1991) 6, 133-151; Francis, G. *et al*, Pharmaceutical Biotechnology Vol. 3. (Borchardt, R. T. ed.); and Stability of Protein Pharmaceuticals: *in vivo* Pathways of Degradation and Strategies for Protein Stabilization (1991) pp 235-263 (Ahem, T. J and Manning, M., ed.s) Plenum, New York].

Attachment of PEG to protein molecules has been achieved using a number of different chemical methods, most of which attach PEG to lysine residues or other amino acid residues on the surface of the protein in a random fashion [Zalipsky, S. & Lee, C. Poly(ethylene glycol) Chemistry: Biotechnical and Biomedical Applications (1992) pp 347-370 (Harris, J. M., ed), Plenum, New York]. This often leads to partial impairment of the function of the protein, for example enzymes have reduced catalytic activity [Nuccl, M. L. *et al ibid*].

Site-specific modification of proteins to introduce sites for PEG attachment has been reported. Interleukin-2, for example, has been modified by mutagenesis to replace a threonine residue which is normally glycosylated by a cysteine to allow attachment of PEG, [Goodson, R. J. & Katre, N. V. Bio/Technology (1990) 8, 343-346]. A site which is normally glycosylated was chosen as this was thought to be capable of tolerating PEG modification without perturbation of the protein structure. In another example, the enzyme purine nucleoside phosphorylase has been modified to selectively replace arginine residues with lysines to provide in this instance up to eighteen additional potential PEG attachment sites per enzyme molecule [Hershfield, M. S. *et al* P.N.A.S. (1991), 88, 7185-7189].

Previous studies with antibodies and antibody fragments have used random PEG attachment via lysine residues [e.g. Ling, T. G. I. & Mattiasson, B. J. Immunol. Methods (1983), 59, 327-337; Wilkinson, I. *et al* Immunol. Letters (1987) 15, 17-22; Kitamura, K. *et al* Cancer Res. (1991), 51, 4310-4315; Delgado, C. *et al* Br. J. Cancer (1996), 73, 175-182] and thiolated derivatives [Pedley, R. B. *et al* Br. J. Cancer (1994), 70, 1126-1130]. Random attachment has often resulted in modified antibodies which are only able to bind their target antigen with reduced affinity, avidity or specificity. In one attempt to overcome this, critical lysine residues in antigen binding (CDR) loops have been replaced with arginines to allow modification with less loss in immunoreactivity [Benhar, I. *et al* Bioconjugate Chemistry (1994) 5, 321-326].

Specific sites in the constant and the hinge regions of antibodies can be engineered to allow site-specific linkage of a range of effector and reporter molecules [Lyons, A. *et al* Prot. Eng. (1990), 3, 703-709; and European Patent Specifications Nos. 348442 and 347433]. We have now determined that site-specific attachment of polymers to monovalent antibody fragments can be used to avoid the loss of immunoreactivity previously associated with random attachment processes. Furthermore, fragments modified in this way have markedly improved binding and/or pharmacokinetic properties when compared to fragments which have been modified randomly with the same number and type of polymer molecules.

Thus according to one aspect of the invention we provide a modified monovalent antibody fragment comprising a monovalent antibody fragment and at least one polymer molecule in covalent linkage characterised in that each cysteine residue located in the antibody fragment outside of the variable region domain of the fragment is either covalently linked through its sulphur atom to a polymer molecule or is in disulphide linkage with a second cysteine residue located in the fragment provided that at least one of said cysteine residues is linked to a polymer molecule.

The modified antibody fragment according to the invention is essentially a monovalent antibody fragment covalently linked to one or more, for example one, two or three polymer molecules through one or more , e.g. one, two or three cysteine residues located in the fragment outside of its variable region domain. The fragment may additionally have one or more effector or reporter molecules covalently attached to it as described hereinafter.

The modified antibody fragment of the invention will in general be capable of selectively binding to an antigen. The antigen may be any cell-associated antigen, for example a cell surface antigen such as a T-cell, endothelial cell or tumour cell marker, or it may be a soluble antigen. Particular examples of cell surface antigens include adhesion molecules, for example integrins such as β1 integrins, e.g. VLA-4, E-selectin, P-selectin or L-selectin, CD2, CD3, CD4, CD5, CD7, CD8, CD11a, CD11b, CD18, CD19, CD20, CD23, CD25, CD33, CD38, CD40, CD45, CDW52, CD69, carcinoembryonic antigen (CEA), human milk fat globulin (HMFG1 and 2), MHC Class I and MHC Class II antigens, and VEGF, and where appropriate, receptors thereof. Soluble antigens include interleukins such as IL-1, IL-2, IL-3, IL-4, IL-5, IL-6, IL-8 or IL-12, viral antigens, for example respiratory syncytial virus or cytomegalovirus antigens, immunoglobulins, such as IgE, interferons such as interferon-α, interferon-β or interferon-γ, tumour necrosis factor-α, tumour necrosis factor-β, colony stimulating factors such as G-CSF or GM-CSF, and platelet derived growth factors such as PDGF-α, and PDGF-β and where appropriate receptors thereof.

The term variable region domain as used herein in relation to the fragment according to the invention is intended to mean that part of the antibody fragment which contains the antigen binding site. The variable region domain may be of any size or amino acid composition and will generally comprise at least one hypervariable amino acid sequence responsible for antigen binding embedded in a framework sequence. In general terms the variable (V) region domain may be any suitable arrangement of immunoglobulin heavy (V_{H}) and/or light (V_{L}) chain variable domains. Thus for example the V region domain may be monomeric and be a V_{H} or V_{L} domain where these are capable of independently binding antigen with acceptable affinity. Alternatively the V region domain may be dimeric and contain V_{H}-V_{H}, V_{H}-V_{L}, or V_{L}-V_{L}, dirners in which the VH and V_{L} chains are non-covalently associated. Where desired, however, the chains may be covalently coupled either directly, for example via a disulphide bond between the two variable domains, or through a linker, for example a peptide linker, to form a single chain domain.

The variable region domain may be any naturally occurring variable domain or an engineered version thereof. By engineered version is meant a variable region domain which has been created using recombinant DNA engineering techniques. Such engineered versions include those created for example from natural antibody variable regions by insertions, deletions or changes in or to the amino acid sequences of the natural antibodies. Particular examples of this type include those engineered variable region domains containing at least one CDR and optionally one or more framework amino acids from one antibody and the remainder of the variable region domain from a second antibody.

The variable region domain will generally be covalently attached to at least one cysteine residue or in particular two or three cysteine residues each covalently linked through its sulphur atom to a polymer molecule.

The location of each cysteine residue may be varied according to the size and nature of the antibody fragment required. Thus, in one extreme example a cysteine residue linked through its sulphur atom to a polymer may be attached directly to a C-terminal amino acid of the variable region domain. This may be for example the C-terminus of a V_{H} or V_{L} chain as described above. If desired, in this example, further amino acids, including further cysteine-linked polymers, may be covalentiy linked to the C-terminus of the first cysteine residue.

In practice however, it is generally preferable that the variable region domain is covalently attached at a C-terminal amino acid to at least one other antibody domain or a fragment thereof which contains, or is attached to one, two, three or more cysteine residues, each covalently linked through its sulphur atom to a polymer molecule. Thus, for example where a V_{H} domain is present in the variable region domain this may be linked to an immunoglobulin C_{H}1 domain or a fragment thereof. Similarly a V_{L} domain may be linked to a C_{K} domain or a fragment thereof. In this way for example the fragment according to the invention may be a Fab fragment wherein the antigen binding domain contains associated V_{H} and V_{L} domains covalently linked at their C-termini to a CH1 and C_{K} domain respectively. The CH1 domain may be extended with further amino acids, for example to provide a hinge region domain as found in a Fab' fragment, or to provide further domains, such as antibody CH2 and CH3 domains. In each of the above cases one or more, e.g. one, two or three, cysteine residues each linked to a polymer molecule may be located at any point throughout any domain.

The polymer molecule in the fragment according to the invention may in general be a synthetic or naturally occurring polymer, for example an optionally substituted straight or branched chain polyalkylene, polyalkenylene or polyoxyalkylene polymer or a branched or unbranched polysaccharide, e.g. a homo- or heteropolysaccharide.

Particular optional substituents which may be present on the above-mentioned synthetic polymers include one or more hydroxy, methyl or methoxy groups. Particular examples of synthetic polymers include optionally substituted straight or branched chain poly(ethylene glycol), poly(propylene glycol), or poly(vinyl alcohol) and derivatives thereof, especially optionally substituted poly(ethylene glycol) such as methoxy(polyethylene glycol) and derivatives thereof. Particular naturally occurring polymers include lactose, amylose, dextran or glycogen and derivatives thereof. "Derivatives" as used herein is intended to include reactive derivatives, for example thiol-selective reactive groups such as maleimides and the like. The reactive group may be linked directly or through a linker segment to the polymer. It will be appreciated that the residue of such a group will in some instances form part of the product of the invention as the linking group between the antibody fragment and the polymer.

The size of the polymer may be varied as desired, but will generally be in an average molecular weight range from around 500Da to around 50000Da for example from 5000 to 40000Da and including 25000 to 40000Da. The polymer size may in particular be selected on the basis of the intended use of the product. Thus for example where the product is intended to leave the circulation and penetrate tissue, for example for use in the treatment of a tumour, it may be advantageous to use a small molecular weight polymer, for example around 5000Da. For applications where the product remains in the circulation it may be advantageous to use a higher molecular weight polymer, for example in the range 25000Da to 40000Da.

As explained above, each polymer molecule in the modified antibody fragment according to the invention is covalently linked to a sulphur atom of a cysteine residue located in the fragment. The covalent linkage will generally be a disulphide bond or, in particular, a sulphur-carbon bond.

Particularly useful fragments according to the invention are those wherein two or especially three cysteine residues located in the fragment outside of the variable region domain is each covalently linked through its sulphur atom to a polymer molecule, any other cysteine residue located in the fragment outside of the variable region domain being in disulphide linkage with a second cysteine residue located in the fragment. In these particular fragments the polymer may especially be a synthetic polymer, particularly a polyalkylene polymer such as poly(ethylene glycol) or especially methoxypoly(ethylene glycol) or a derivative thereof, and especially with a molecular weight in the range from about 25000Da to about 40000Da.

Where desired, the antibody fragment according to the invention may have one or more effector or reporter molecules attached to it and the invention extends to such modified antibodies. The effector or reporter molecules may be attached to the antibody fragment through any available amino acid side-chain or terminal amino acid functional group located in the fragment, for example any free amino, imino, hydroxyl or carboxyl group.

Effector molecules include, for example, antineoplastic agents, toxins (such as enzymatically active toxins of bacterial or plant origin and fragments thereof e.g. ricin and fragments thereof) biologically active proteins, for example enzymes, nucleic acids and fragments thereof, e.g. DNA, RNA and fragments thereof, radionuclides, particularly radioiodide, and chelated metals. Suitable reporter groups include chelated metals, fluorescent compounds or compounds which may be detected by NMR or ESR spectroscopy.

Particular antineoplastic agents include cytotoxic and cytostatic agents, for example atkylating agents, such as nitrogen mustards (e.g. chlorambucil, melphalan, mechlorethamine, cyclophosphamide, or uracil mustard) and derivatives thereof, triethylenephosphoramide, triethylenethiophosphoramide, busulphan, or cisplatin; antimetabolites, such as methotrexate, fluorouracil, floxuridine, cytarabine, mercaptopurine, thioguanine, fluoroacetic acid or fluorocitric acid, antibiotics, such as bleomycins (e.g. bleomycin sulphate), doxorubicin, daunorubicin, mitomycins (e.g. mitomycin C), actinomycins (e.g. dactinomycin) plicamycin, calichaemicin and derivatives thereof, or esperamicin and derivatives thereof; mitotic inhibitors, such as etoposide, vincristine or vinblastine and derivatives thereof; alkaloids, such as ellipticine; polyols such as taxicin-I or taxicin-II; hormones, such as androgens (e.g. dromostanolone or testotactone), progestins (e.g. megestrol acetate or medroxyprogesterone acetate), estrogens (e.g. dimethylstilbestrol diphosphate, polyestradiol phosphate or estramustine phosphate) or antiestrogens (e.g. tamoxifen); anthraquinones, such as mitoxantrone, ureas, such as hydroxyurea; hydrazines, such as procarbazine; or imidazoles, such as dacarbazine.

Particularly useful effector groups are calichaemicin and derivatives thereof (see for example South African Patent Specifications Nos. 85/8794, 88/8127 and 90/2839).

Chelated metals include chelates of di-or tripositive metals having a coordination number from 2 to 8 inclusive. Particular examples of such metals include technetium (Tc), rhenium (Re), cobalt (Co), copper (Cu), gold (Au), silver (Ag), lead (Pb), bismuth (Bi), indium (In), gallium (Ga), yttrium (Y), terbium (Tb), gadolinium (Gd), and scandium (Sc). In general the metal is preferably a radionuclide. Particular radionudides include ^{99m}Tc, ¹⁸⁶Re, ¹⁸⁸Re, ⁵⁸Co, ⁶⁰Co, ⁶⁷Cu, ¹⁹⁵Au, ¹⁹⁹Au, ¹¹⁰Ag, ²⁰³Pb, ²⁰⁶Bi, ²⁰⁷Bi, ¹¹¹In, ⁶⁷Ga, ⁶⁸Ga, ⁸⁸Y, ⁹⁰Y, ¹⁶⁰Tb, ¹⁵³Gd and ⁴⁷Sc.

The chelated metal may be for example one of the above types of metal chelated with any suitable polydentate chelating agent, for example acyclic or cyclic polyamines, polyethers, (e.g. crown ethers and derivatives thereof); polyamides; porphyrins; and carbocyclic derivatives.

In general, the type of chelating agent will depend on the metal in use. One particularly useful group of chelating agents in conjugates according to the invention, however, are acyclic and cyclic polyamines, especially polyaminocarboxylic acids, for example diethylenetriaminepentaacetic acid and derivatives thereof, and macrocyclic amines, e.g. cyclic tri-aza and tetra-aza derivatives (for example as described in International Patent Specification No. WO 92/22583); and polyamides, especially desferrioxamine and derivatives thereof.

The modified antbody fragment according to the invention may be prepared by reacting an antibody fragment containing at least one reactive cysteine residue with a thiol-selective activated polymer. The reaction may generally be performed in a solvent, for example an aqueous buffer solution such as an acetate or phosphate buffer, at around neutral pH, for example around pH 4.5 to around pH 8.0, at for example ambient temperature. The activated polymer will generally be employed in excess concentration relative to the concentration of the antibody fragment. In some instances it may be necessary to reduce the antibody starting material with a reagent such as β-mercaptoethylamine (for example as described in Example 1 hereinafter) to generate an appropriately reactive cysteine residue. Where necessary, the desired product containing the desired number of polymer molecules may be separated from any other product generated during the production process and containing an unwanted number of polymer molecules by conventional means, for example by chromatography.

The antibody fragment starting material may be obtained from any whole antibody, especially a whole monoclonal antibody, [prepared by conventional immunisation and cell fusion procedures], using any suitable standard enzymatic cleavage and/or digestion techniques, for example by treatment with pepsin. Alternatively, the antibody starting material may be prepared by the use of recombinant DNA techniques involving the manipulation and re-expression of DNA encoding antibody variable and/or constant regions. Such DNA is known and/or is readily available from DNA libraries including for example phage-antibody libraries [see Chiswell, D J and McCafferty, J. Tibtech. 10 80-84 (1992)] or where desired can be synthesised. Standard molecular biology and/or chemistry procedures may be used to sequence and manipulate the DNA, for example, to introduce codons to create cysteine residues, to modify, add or delete other amino acids or domains as desired.

From here, one or more replicable expression vectors containing the DNA may be prepared and used to transform an appropriate cell line, e.g. a non-producing myeloma cell line, such as a mouse NSO line or a bacterial, e.g. *E.coli* line, in which production of the antibody will occur. In order to obtain efficient transcription and translation, the DNA sequence in each vector should include appropriate regulatory sequences, particularly a promoter and leader sequence operably linked to the variable domain sequence. Particular methods for producing antibodies in this way are generally well known and routinely used. For example, basic molecular biology procedures are described by Maniatis *et al* [Molecular Cloning, Cold Spring Harbor Laboratory, New York, 1989]; DNA sequencing can be performed as described in Sanger *et al* [PNAS 74, 5463, (1977)] and the Amersham International plc sequencing handbook; and site directed mutagenesis can be carried out according to the method of Kramer *et al* [Nucl. Acids Res. 12, 9441, (1984)] and the Anglian Biotechnology Ltd handbook. Additionally, there are numerous publications, including patent specifications, detailing techniques suitable for the preparation of antibodies by manipulation of DNA, creation of expression vectors and transformation of appropriate cells, for example as reviewed by Mountain A and Adair, J R in Biotechnology and Genetic Engineering Reviews [ed. Tombs, M P, 10, Chapter 1, 1992, Intercept, Andover, UK] and in International Patent Specification No. WO 91/09967.

The activated polymer starting material for use in the preparation of antibody fragments according to the invention may be any polymer containing a thiol reactive group such as an α-halocarboxylic acid or ester, e.g. iodoacetamide, an imide, e.g. maleimide, a vinyl sulphone, or a disulphide. Such starting materials may be obtained commercially (for example from Shearwater Polymers Inc., Huntsville, AL, USA) or may be prepared from commercially available starting materials using conventional chemical procedures, for example as described by Zalipsky, S & Lee, C, *ibid* and in the Examples hereinafter.

Where it is desired to obtain an antibody fragment according to the invention linked to an effector or reporter molecule this may be prepared by standard chemical or recombinant DNA procedures in which the antibody fragment is linked either directly or via a coupling agent to the effector or reporter molecule either before or after reaction with the activated polymer as appropriate. Particular chemical procedures include for example those described in International Patent Specification Nos. WO 93/06231, WO 92/22583, WO 90,09195 and WO 89/01476. Alternatively, where the effector or reporter molecule is a protein or polypeptide the linkage may be achieved using recombinant DNA procedures, for example as described in International Patent Specification No. WO 86/01533 and European Patent Specification No. 392745.

The antibody fragment according to the invention may be useful in the detection or treatment of a number of diseases or disorders. Such diseases or disorders may include those described under the general headings of infectious disease, e.g. viral infection; inflammatory disease/autoimmunity e.g. rheumatoid arthritis, osteoarthritis, inflammatory bowel disease; cancer; allergic/atopic disease e.g. asthma, eczema; congenital disease, e.g. cystic fibrosis, sickle cell anaemia; dermatologic disease, e.g. psoriasis; neurologic disease, e.g. multiple sclerosis; transplants e.g. organ transplant rejection, graft-versus-host disease; and metabolic/idiopathic disease e.g. diabetes.

The modified antibody fragments according to the invention may be formulated for use in therapy and/or diagnosis and according to a further aspect of the invention we provide a pharmaceutical composition comprising a modified monovalent antibody fragment comprising a monovalent antibody fragment and at least one polymer molecule in covalent linkage characterised in that each covalent linkage is through a sulphur atom of a cysteine residue located in the antibody fragment outside of the variable region domain of the fragment, together with one or more pharmaceutically acceptable excipients, diluents or carriers.

As explained above, the modified antibody fragment in this aspect of the invention may be optionally linked to one or more effector or reporter groups.

The pharmaceutical composition may take any suitable form for administration, and, preferably is in a form suitable for parenteral administration e.g. by injection or infusion, for example by bolus injection or continuous infusion. Where the composition is for injection of infusion, it may take the form of a suspension, solution or emulsion in an oily or aqueous vehicle and it may contain formulatory agents such as suspending, preservative, stabilising and/or dispersing agents.

Alternatively, the antibody composition may be in dry form, for reconstitution before use with an appropriate sterile liquid.

If the antibody composition is suitable for oral administration the formulation may contain, in addition to the active ingredient, additives such as: starch e.g. potato, maize or wheat starch or cellulose or starch derivatives such as microcrystalline cellulose; silica; various sugars such as lactose; magnesium carbonate and/or calcium phosphate. It is desirable that, if the formulation is for oral administration it will be well tolerated by the patient's digestive system. To this end, it may be desirable to include in the formulation mucus formers and resins. It may also be desirable to improve tolerance by formulating the antibody in a capsule which is insoluble in the gastric juices. It may also be preferable to include the antibody or composition in a controlled release formulation.

If the antibody composition is suitable for rectal administration the formulation may contain a binding and/or lubricating agent; for example polymeric glycols, gelatins, cocoa-butter or other vegetable waxes or fats.

Therapeutic and diagnostic uses of fragments according to the invention typically comprise administering an effective amount of the antibody fragment to a human subject. The exact amount to be administered will vary according to the use of the antibody and on the age, sex and condition of the patient but may typically be varied from about 0.1 mg to 1000mg for example from about 1mg to 500mg. The antibody may be administered as a single dose or in a continuous manner over a period of time. Doses may be repeated as appropriate. Typical doses may be for example between 0.1-50mg/kg body weight per single therapeutic dose, particularly between 0.1-20 mg/kg body weight for a single therapeutic dose.

The following Examples illustrate the invention. In the Examples, the following antibody fragments are used and are identified in each case by the abbreviated name given below. In each instance the antibody was prepared from a mouse monoclonal antibody as described in International Patent Specification No. WO92/01059 (A5B7) or by using similar methods (hTNF40 and cTN3):
- hA5B7: - This is an engineered human antibody which recognises carcinoembryonic antigen. The antibody fragment used here has one cysteine residue available for pegylation and located in its hinge region after activation with β-mercaptoethylamine.
- hTNF40: - This is an engineered human antibody which recognises human TNFα. Two hTNF40 antibody fragments are used in the Examples, one (Example 2) which has a single cysteine residue in the hinge region (see hA5B7 above), and a second (Example 3) which has two hinge cysteine residues, available for pegylation.
- cTN3: - This is a chimeric hamster/mouse antibody which recognises mouse TNFα and has a mouse IgG2a constant region. The antibody has three hinge cysteine residues available for pegylation.
- hg162: - This is an engineered human antibody which recognises human PDGFβ receptor. The antibody fragment used here has a single cysteine residue present in its hinge region available for pegylation.

The following abbreviations are used in Example 1:

PEG = CH₃O(CH₂CH₂O)ₙ(CH₂)₂NHCO(CH₂)₂-

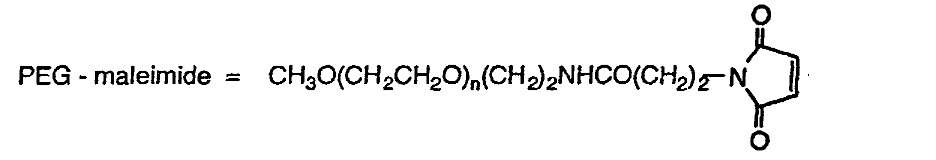

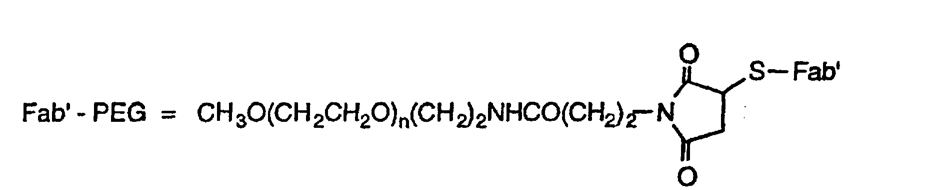

In Examples 2-7, the PEG abbreviation is used to refer to straight or branched methoxypoly(ethylene glycol), with or without a linker segment between the poly(ethylene glycol) chain and thiol reactive group as indicated. In each Example, linkage to the antibody occurs either through a -S-C- bond as described above, or, in Example 5 through, a -S-S-bond.

In all the Examples, the following abbreviations are used:
- DTDP: -4,4'-dithiodipyridine
- PBS: - phosphate buffered saline
- HPLC: -high performance liquid chromatography
- AUC: - area under the curve

### EXAMPLE 1

### Purification of hA5B7 Fab'

hA5B7 Fab' was expressed in *E.coli* W3110 cells grown in a 1.5 litre fermenter. Cells were harvested by centrifugation and resuspended to the original volume with 100mM Tris pH 7.4 containing 10mM EDTA, and incubated overnight at 55°C. The resulting cell extract was then clarified by centrifugation, made 1M with respect to glycine, and the pH adjusted to 7.5 with 50% (w/v) sodium glycinate. This sample was applied to a column of Streamline® A (Pharmacia) equilibrated with 1M glycine/glycinate pH8.0. After washing with equilibration buffer, hA5B7 Fab' was eluted with 0.1M citrate pH3.0. The eluted hA5B7 Fab' was then adjusted to pH6.0 with 2M Tris pH8.5 and concentrated by ultrafiltration.

### Preparation of PEG-maleimide reagent

A maleimide derivative of PEG was prepared as previously described [Pedley *et al* (1994)*ibid*]. Methoxypolyoxyethylene amine (average molecular weight approximately 5000, Sigma) was dissolved in 0.1M sodium phosphate buffer, pH7.0, and incubated with a 1.2-fold molar excess of 3-maleimido-propionic acid N-hydroxysuccinimide ester for 1 h at room temperature. The extent of reaction was determined by spotting aliquots of the reaction mixture onto a TLC plate (Kieselgel 60), and developing with ninhydrin. The reaction was considered complete when there was no purple coloration remaining (amine reaction with ninhydrin). The PEG-maleimide product was desalted using a Sephadex G-25 (PD-10) column (Pharmacia) into deionised water, and lyophilised. The presence of active maleimide groups was demonstrated by back titration with β-mercaptoethylamine.

### Preparation of hA5B7 Fab'-PEG (site-specific)

Purified hA5B7 Fab' at a concentration of approximately 11mg/ml was desalted into 0.1M acetate buffer, pH6.0, using a Bio-Spin 6 column (BioRad). The hinge thiol group was then activated by reduction with β-mercaptoethylamine. hA5B7 Fab' was incubated with 5mM β-mercaptoethylamine in 0.1M acetate buffer pH6.0 for 30 min at 37°C. The sample was then desalted using a Bio-Spin 6 column into 0.1M phosphate buffer pH6.0. The number of thiol groups per Fab' molecule was measured by titration with DTDP as previously described [Lyons *et al* (1990) *ibid*]. The sample was then incubated for 2.5h at room temperature with a greater than 10-fold molar excess of PEG-maleimide produced as described above. The PEG modified Fab' was then desalted on a Bio-Spin 6 column into phosphate buffered saline pH 6.8, and a thiol titration carried out to ensure that the thiol groups had reacted fully with the PEG-maleimide reagent.

### Preparation of Randomly Modified hA5B7 Fab'-PEG

hA5B7 Fab' at approximately 11mg/ml was desalted into 0.1M phosphate pH8.0, using a Bio-Spin 6 column. Thiols were introduced randomly onto lysine residues by reaction with a 7-fold molar excess of 2-iminothiolane (Traut's reagent) for 1h at room temperature. After desalting into 0.1M phosphate pH6.0 using a Bio-Spin 6 column, the number of thiol groups introduced was determined using titration with DTDP. The thiolated hA5B7 Fab' was then reacted with PEG-maleimide and desalted as described above for the site-specific modification.

### Analysis of PEG-modified samples

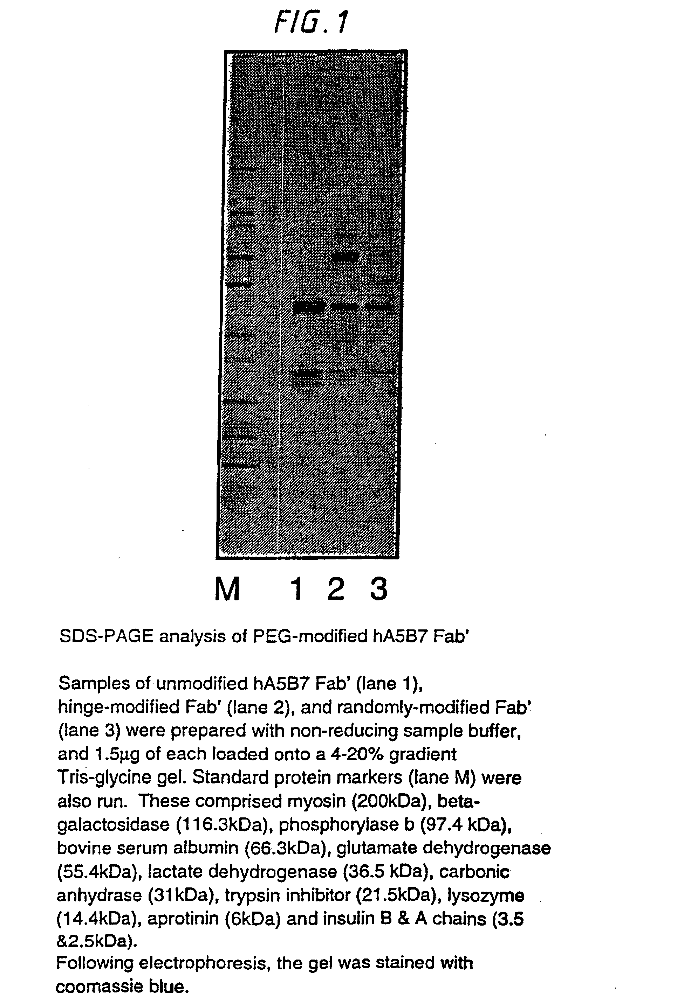

In order to compare the two methods of PEG attachment (site-specific and random), samples with a similar degree of modification were sought. Using the conditions described above, attachment site-specifically at the hinge resulted in an average of 0.98 PEG molecules per Fab' molecule, whilst the random attachment via Traut's reagent resulted in an average of 1.18 PEG molecules per Fab'. Analysis by SDS-PAGE under non-reducing conditions (Figure 1) revealed that the major band in the unmodified hA5B7 Fab' sample (lane 1) has a molecular weight of about 50kDa as expected. The sample with PEG attached via site-specific means at the hinge (lane 2) contains some residual unmodified Fab, as well as two other distinct species with larger sizes, the most prominent of which has an apparent molecular weight of approximately 66Dka. A similar amount of residual unreacted Fab' is also detected in the randomly modified sample. This sample can be seen to be much more heterogeneous than the site-specifically modified sample with some discrete bands but also a diffuse staining of bands coveting a relatively wide molecular weight range. The exact size of PEG-modified proteins cannot be deduced from this technique since the attachment of PEG is known to alter the running of protein bands on electrophoresis relative to standard proteins. However, it can be seen that both preparations have been modified with PEG and that different molecular species are produced in each case.

In order to assess the effect of PEG modification on the activity of hA5B7 Fab', a kinetic assay was carried out by surface plasmon resonance using a Biacore 2000 instrument (Pharmacia Biosensor). The assay was carried out by a modification of the method described previously [Abraham *et al* J. Immunol. Methods (1995), 183, 119-125].

Carcinoembryonic antigen (CEA) (100ml, 0.92µg/ml) was buffer exchanged into 0.1M sodium acetate pH 5.5 using a BioSpin 6 column (BioRad). Sodium periodate (2µl, 50mM in 0.1M sodium acetate, pH 5.5 freshly prepared) was added and the mixture was incubated on ice for 20 minutes. The reaction was stopped by buffer exchange into 10mM sodium acetate pH 4.0 on a BioSpin 6 column to give 100µl oxidised CEA at approximately 0.89mg/ml. The oxidised CEA was then immobilized onto a CM5 sensor chip (Pharmacia Biosensor) using the standard aldehyde coupling procedure described in the manufacturers instructions. Briefly, this involved activation of the surface by injection of EDC/NHS reagent (15µl, amine coupling kit, Pharmacia Biosensor) at a flow-rate of 5µl/min, followed by injection of 35µl 5mM hydrazine and then 35µl 1M ethanolamine. This was followed by injection of 35µl oxidised CEA at either 5, 1, 0.2 or 0.05µg/ml in 10mM sodium acetate pH 4.0. Finally 40µl 0.1M sodium cyanoborohydride was passed over the surface and the sensorchip washed with 4 successive aliquotes of 10mM hydrochloric acid prior to use. Each hA5B7 Fab' sample was diluted in eight doubling dilutions from 20µg/ml in HBS buffer (Pharmacia Biosensor). The samples were injected over the CEA surface to observe binding and dissociation kinetics. Association and dissociation rate constants were calculated by assuming simple 1:1 binding kinetics and applying the non-linear rate equations supplied with the manufacturers analysis program.

The results of this analysis (Table 1) show that in this assay there was some loss of potency (to 56% of the unmodified hA5B7 Fab') as a result of the site-specific attachment at the hinge. However, the attachment of a similar number of PEG molecules in a random fashion resulted in material with only 29% immunoreactivity. The loss of potency appears to be mainly due to a reduced rate of association, although there may also, be a slightly increased dissociation rate.

For pharmacokinetic analysis Fab' samples were labelled with ¹²⁵I using Bolton-Hunter reagent by standard methodology and desalted into phosphate buffered saline pH6.8 to remove unreacted ¹²⁵I. Groups of six male Wistar rats were injected i.v. into the tail vein with 20µg of labelled Fab'. At selected time points, blood samples were taken, counted in a gamma counter, and the percent injected dose per gram of blood calculated. The clearance rates and area under the curve values were determined using the SIPHAR software package.

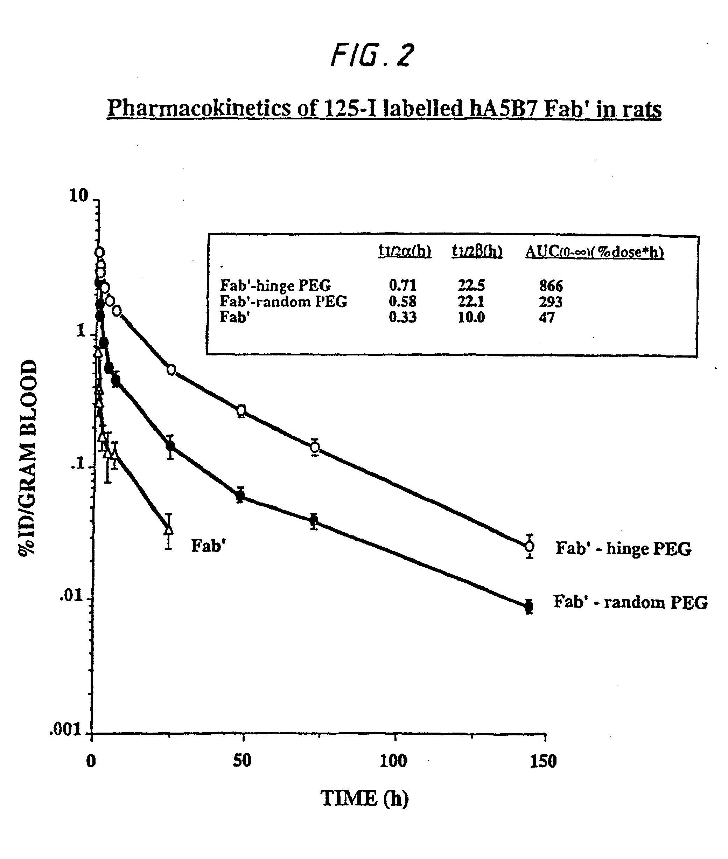

Clearance curves for hA5B7 Fab' and the two PEG modified preparations are shown in Figure 2. From these curves it can be clearly seen that the clearance of PEG-modified hA5B7 Fab' is significantly slower than that of the unmodified hA5B7 Fab'. In addition, the clearance of the site-specifically modified Fab' is unexpectedly slower than that of the randomly modified material. Calculated pharmacokinetic parameters (Table 2) show that modification with PEG decreases the rate of clearance of hA5B7 Fab' in both the α and β phases by approximately two-fold. These improvements are reflected in the area under the curve (AUC) values which show a significant effect of PEG attachment. The attachment of PEG site-specifically results in an increase of the AUC of approximately 18-fold compared to the unmodified Fab', whilst randomly attached PEG results in only a six-fold increase compared to the unmodified Fab'.

**Table 1**

| Immunoreactivity of hA5B7 Fab' samples binding to CEA by BlAcore analysis. | | | | |
|---|---|---|---|---|
| | kₐₛₛ (x10⁴ M⁻¹ s⁻¹) | k_{diss} (x10⁻⁴s⁻¹) | K_{D} (nM) | Immuno-reactivity |
| Unmodified Fab' | 8.60 | 1.61 | 1.87 | 100% |
| Hinge attachment | 5.18 | 1.73 | 3.34 | 56% |
| Random attachment | 3.26 | 2.12 | 6.50 | 29% |

**Table 2**

| Pharmacokinetic properties of PEG-modified Fabs | | | |
|---|---|---|---|
| | t1/2α (h) | t1/2β (h) | AUC (0-∞) (% dose x h) |
| Unmodified Fab' | 0.33 | 10.0 | 47 |
| Random attachment | 0.58 | 22.1 | 293 |
| Hinge attachment | 0.71 | 22.5 | 866 |

### EXAMPLE 2

### Site-specific attachment of PEG to hTNF40 Fab'

### Purification of hTNF40 Fab'

hTNF40 Fab' was expressed in *E. coli* W3110 cells grown in a 1.5 litre fermenter and a cell extract prepared as described in Example 1. The cell extract was diluted to a conductivity of 3.5µS/cm, adjusted to pH4.5 and applied to a column of Streamline® SP (Pharmacia) equilibrated with 50mM acetate buffer pH4.5. After washing with equilibration buffer, the Fab' was eluted with 200mM sodium chloride in 50mM acetate buffer pH4.5. The pH of the eluted material was adjusted to 6 and the Fab' was purified further by applying to a column of protein G-sepharose equilibrated in PBS. After washing with PBS, the Fab' was eluted wih 0.1M glycine-hydrochloric acid pH2.7 and immediately the pH was readjusted to 6. The purified Fab' was then concentrated to >10mg/ml by ultrafiltration.

### Preparation of hTNF40 Fab'-PEG(25kDa) and hTNF40 Fab'-PEG(40kDa) through hinge thiol PEG attachment

Purified Fab' was buffer exchanged into 0.1M phosphate buffer pH6 containing 2mM EDTA. The hinge thiol group was then activated by reduction with 5mM β-mercaptoethylamine as described in Example 1. This resulted in an average of 1.1 thiols per Fab' as determined by titration with DTDP. Fab'-PEG samples were then produced with both 25kDa and a 40kDa PEG using PEG-maleimide derivatives supplied by Shearwater Polymers Inc. Huntsville, AL, USA. The 25kDa PEG-maleimide derivative was a single PEG chain linked directly to a maleimide group, and the 40kDa PEG-maleimide derivative was prepared from a branched structure comprising two 20kDa PEG chains linked through a lysine derivative to a maleimide group. Freshly reduced and desalted Fab' was incubated with a three-fold molar excess of 25kDa PEG-maleimide or a nine-fold molar excess of 40kDa PEG-maleimide overnight at room temperature and the resulting Fab'-PEG conjugates purified by gel-filtration HPLC using a Zorbax GF-250 column run in 0.2M phosphate buffer pH7.0. For comparative purposes, a randomly PEG-modified hTNF40 Fab' was also prepared with 25kDa PEG as described for 5kDa PEG attachment to A5B7 Fab' in Example 1. Conjugate with an average of 1.5 PEG molecules per Fab' was prepared.

### Analysis of Fab'-PEG conjugates

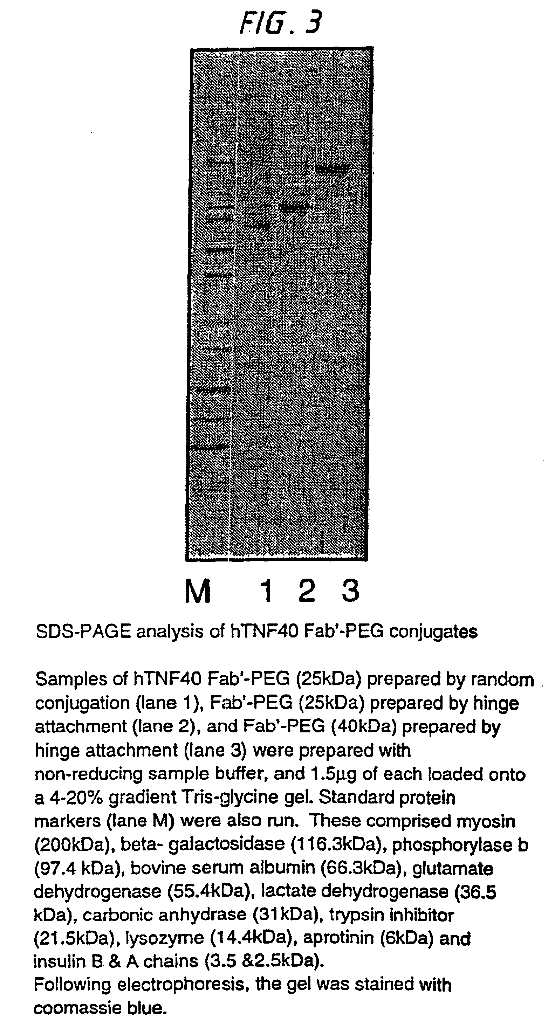

Purified samples were examined by SDS-PAGE under non-reducing conditions. PEG conjugates ran as expected with slower mobility than the unmodified Fab' and were shown to be free of unmodified Fab' (Figure 3). In addition, the hinge modified Fab'-PEG conjugates were more defined on SDS-PAGE than the random PEG conjugate.

The ability of the PEG conjugates to bind to their antigen, TNF, was examined in an L929 bioassay and compared to both the unmodified Fab' and IgG. L929 is an adherent mouse fibroblast cell line which is sensitive to the cytotoxic action of TNF in the presence of the protein synthesis inhibitor actinomycin D. It is therefore possible to compare the activity of TNF antagonists such as anti-TNF antibodies using this cell line. 96 well plates seeded with L929 cell monolayers are cultured in 100ng/ml of human TNF with 1 µg/ml actinomycin D for 18 hours in RPMI medium supplemented with glutamine. Under these conditions between 95-100% of the cells are killed and cease to adhere to tissue culture plastic. The remaining cells were then fixed with 100% methanol for 1 minute and stained with 5% crystal violet. Plates were then washed and the stained cells dissolved in 30% acetic acid before analysis on a plate reader. This experiment was also carried out with titrations of antibody samples added to the wells at the same time as the TNF. Results are plotted as TNF antagonist concentration against residual TNF where the lower the TNF concentration the greater the inhibition. Inhibitory antibodies can then be compared by calculating the concentration of each required to inhibit 90% of the TNF activity to give an IC90 value.

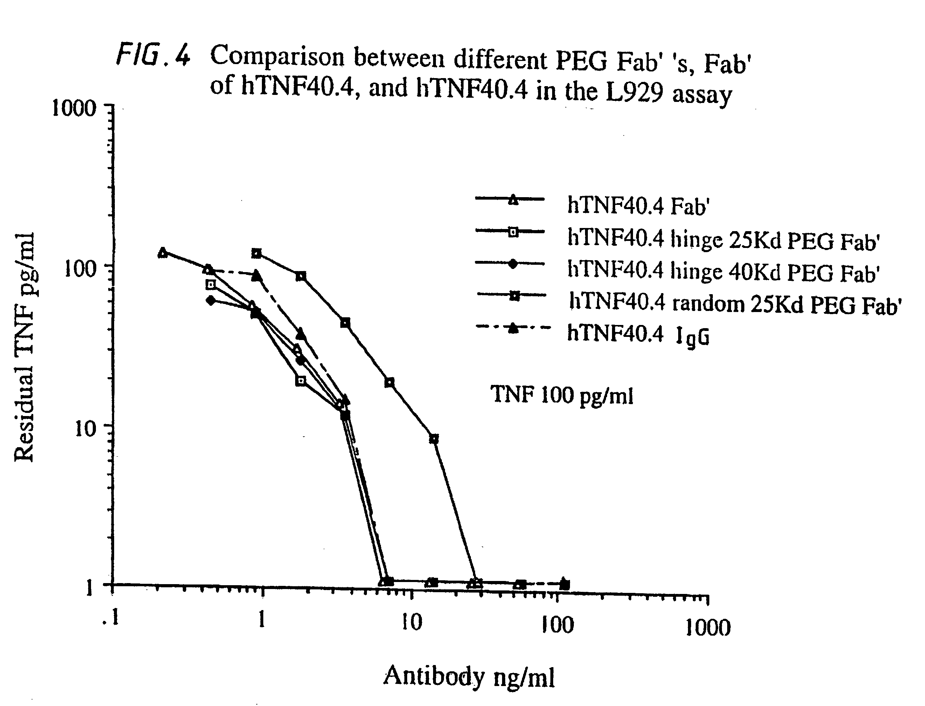

The hTNF40 antibody and its Fab' fragment have an IC90 of 3ng/ml in this L929 assay. Results with both 25kDa and 40kDa hinge modified PEG conjugates also showed IC90 values of 3ng/ml, suggesting that these conjugates neutralised TNF to the same extent as hTNF40 Fab' and IgG (Figure 4). Randomly conjugated hTNF40 Fab'-PEG was less potent than the hinge conjugated preparations with an IC90 value of 10ng/ml.

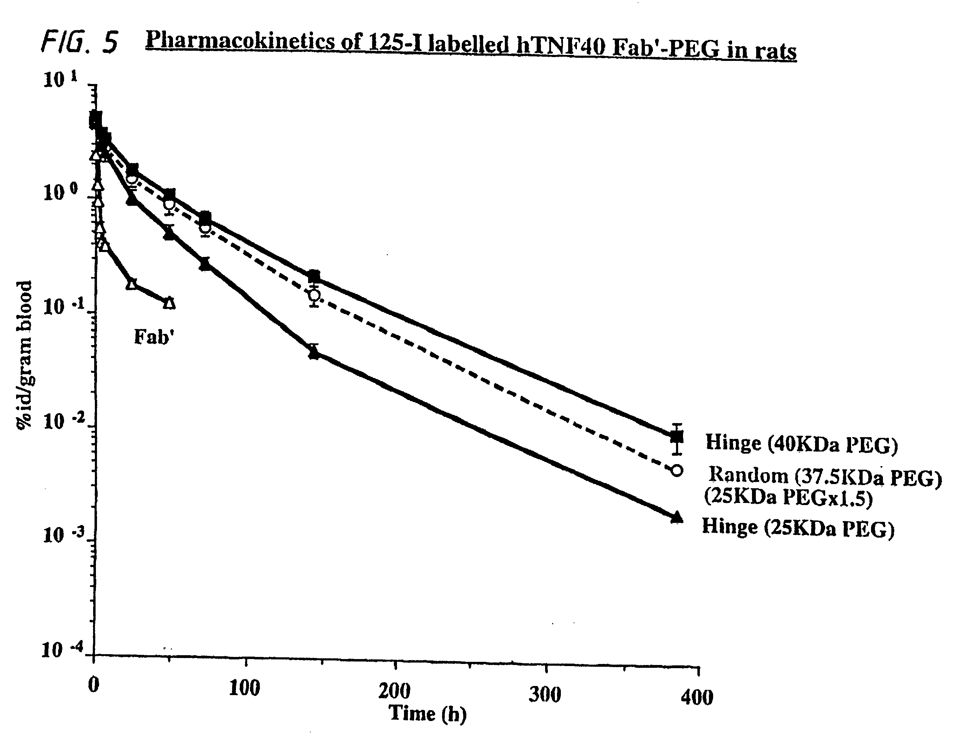

Pharmacokinetic analysis of conjugates was performed in rats with ¹²⁵I labelled material as described in Example 1. The results, (Figure 5) demonstrate increased circulating half-life for all PEG modified Fab' fragments compared to the unmodified Fab'. Attachment of larger PEG molecules at the hinge region increased circulating half-life more than smaller PEG molecules. Randomly modified Fab' with an average of 1.5 25kDa PEG molecules per Fab' (average 37.5kDa PEG per Fab') showed an intermediate circulating half-life between the hinge region Fab' conjugates with 25kDa and 40kDa PEG.

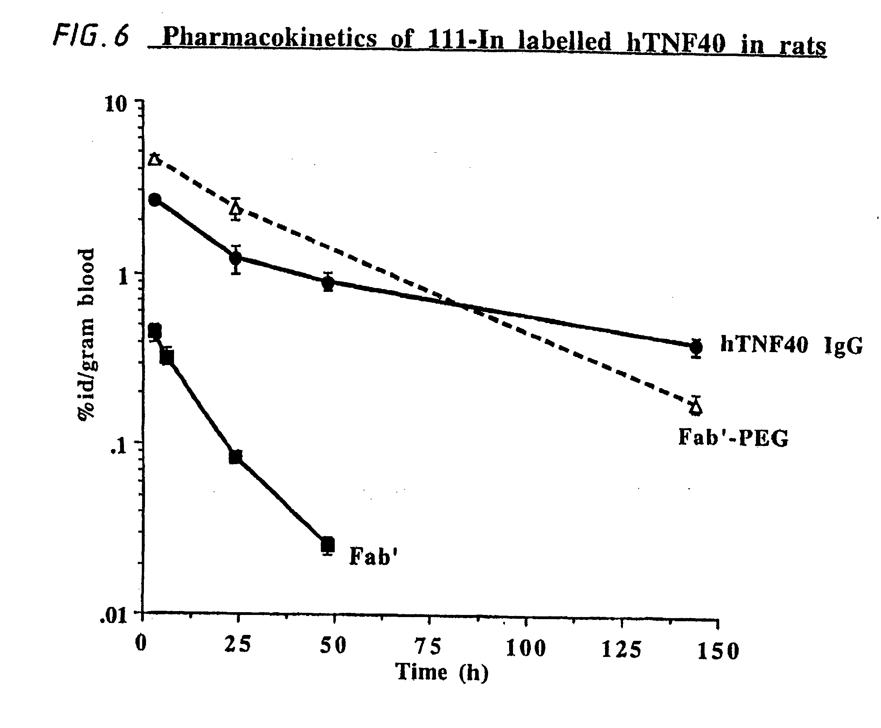

In a separate experiment the pharmacokinetics of hTNF40 IgG, Fab' and Fab'-PEG (25kDa hinge attached) were compared in rats after labelling with ¹¹¹In. IgG and Fab' were conjugated to a 9N3 macrocyclic chelator for labelling with ¹¹¹In as described [Turner *et al*., Br. J. Cancer 70, 35-41 (1994)]. For the PEG conjugate, Fab'-9N3 conjugate was prepared and labelled with ¹¹¹In using the same method and subsequently 25kDa PEG was attached to the hinge region as described above.The labelled conjugate was then purified by gel-filtration HPLC. Results of the rat pharmacokinetic experiment with these ¹¹¹In labelled conjugates (Figure 6) again demonstrate an increased half-life in circulation for the PEG modified Fab' compared to unmodified Fab' with blood levels higher than IgG by 144 hours.

### EXAMPLE 3

### Production of hTNF40 Fab'-(PEG)₂

In this example the applicability of this method to Fab' fragments produced by digestion from IgG which contain two hinge cysteine residues is demonstrated.

### Preparation of hTNF40 Fab'-(PEG)₂

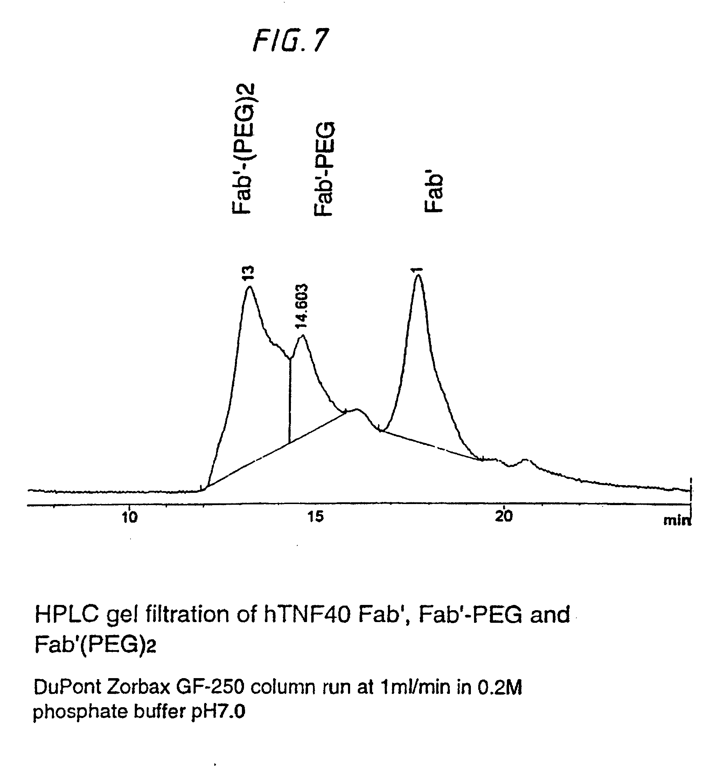

hTNF40 whole antibody was expressed in NSO cells, purified by protein A sepharose chromatography and F(ab')₂ produced by digestion with pepsin using standard techniques. F(ab')₂ was purified by gel filtration chromatography using Sephacryl S-200 HR. After buffer exchange into 0.1M phosphate pH8 containing 5mM EDTA, F(ab')₂ was reduced to Fab' by incubation with 5mM β-mercaptoethylamine for 30 minutes at 37°C. PEG-maleimide (25kDa PEG - see Example 2) was then added to 3 fold molar excess over Fab' concentration and the reaction allowed to proceed overnight. The resulting mixture was analysed by gel-filtration HPLC and found to contain a mixture of unmodified Fab', Fab'-PEG and Fab'-(PEG)₂ (Figure 7). The PEG modifed material was purified by gel-filtration HPLC and resulted in a mixture of 1:1.2, Fab'-PEG : Fab'-(PEG)₂.

### Antigen binding and pharmacokinetic analysis

Antigen binding activity was assessed by BlAcore assay which measured affinity for TNF binding. Fab' or PEG modified samples were captured with an immobilized anti-Fab' antibody and human TNF passed over the surface. The kinetics of TNF binding were then analysed. Results suggested that the mixture of Fab'-PEG and Fab'-(PEG)₂ had a binding affinity, K_{D}, of 0.14nM. This compared favourably to the unmodified Fab' which had a K_{D} of 0.28nM in the same assay, suggesting that there was no loss of antigen binding activity due to PEG attachment.

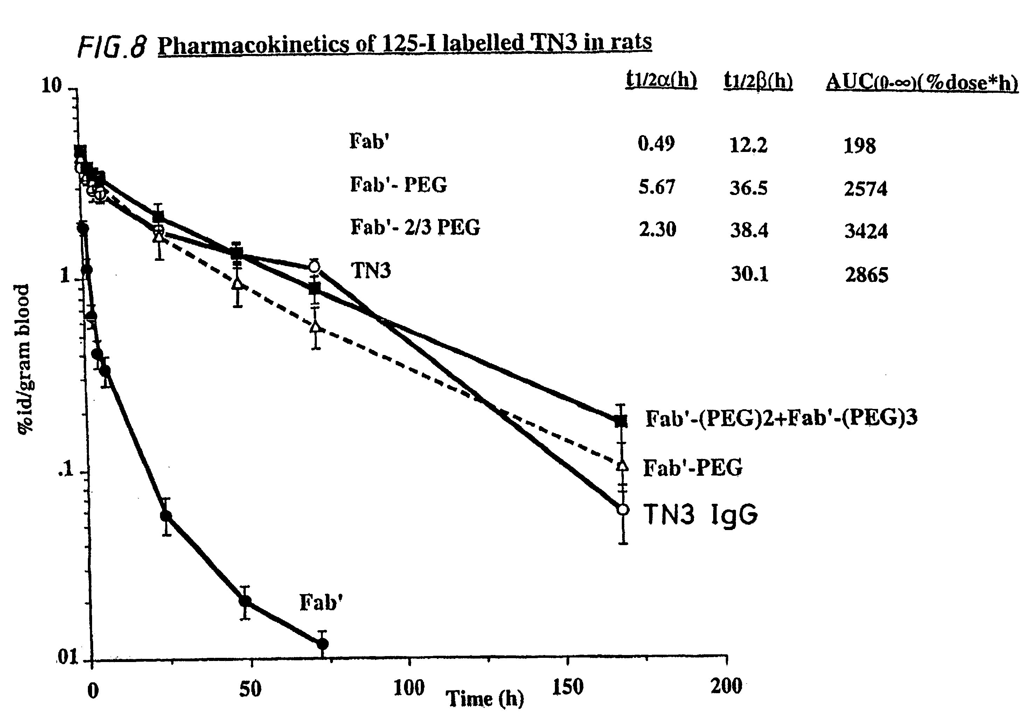

Pharmacokinetics of the purifed material containing a mixture of Fab'-PEG and Fab'-(PEG)₂ were assessed in rats after radiolabelling with ¹²⁵I. Results (Figure 8) demonstrate an improved circulating half-life compared to Fab'-PEG alone which had been produced from *E. coil* expressed material with a single hinge cysteine prepared as described in Example 2.

### EXAMPLE 4

### Preparation of cTN3 Fab'-PEG, Fab'-(PEG)₂ and Fab'-(PEG)₃

cTN3 antibody was expressed in NSO cells, purified and digested with pepsin to produce F(ab')₂ using standard techniques. Purified F(ab')₂ was buffer exchanged into 0.1M phosphate buffer pH8 containing 5mM EDTA and then reduced with 9mM β-mercaptoethylamine and modified with PEG at the hinge region as described in Example 3. A mixture of Fab', Fab'-PEG, Fab'-(PEG)₂ and Fab'-(PEG)₃ was obtained. Fab' and Fab'-PEG was separated from Fab'-(PEG)₂ and Fab'-(PEG)₃ by gel-filtration HPLC. The purifed sample of Fab'-(PEG)₂ and Fab'-(PEG)₃ contained a ratio of 1.6:1 Fab'-(PEG)₂ : Fab'-(PEG)₃.

### Antigen binding and pharmacokinetic analysis

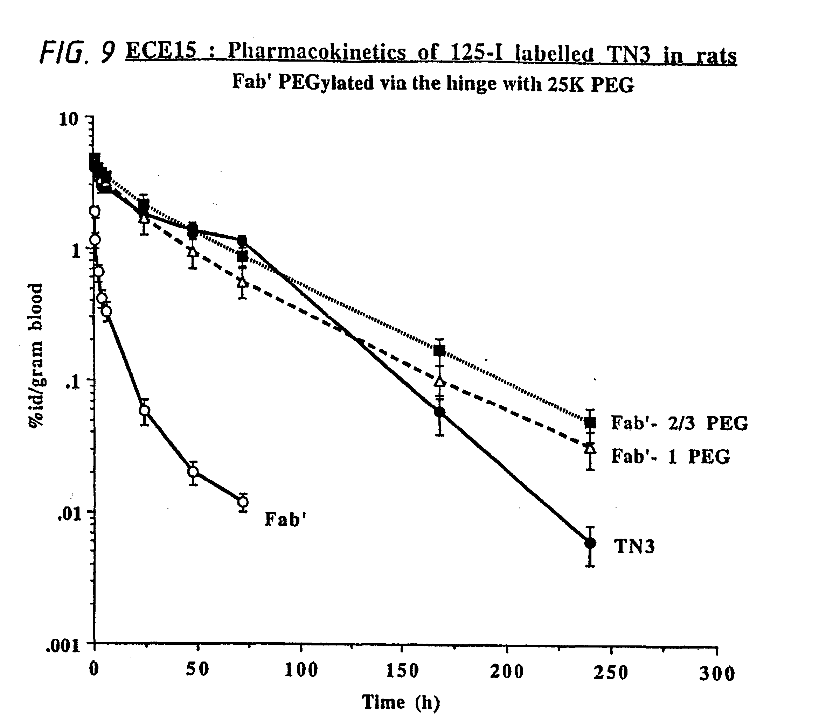

Antigen binding analysis of cTN3 Fab', Fab'-PEG, and Fab'-(PEG)₂ + Fab'-(PEG)₃ was carried out in a variant of the L929 bioassay described above. The TN3 antibodies were incubated with mouse TNF. In this assay cTN3 IgG has an IC90 of approximately 1000pg/ml. In the same assay both the Fab' and Fab'-PEG have the same inhibition profile and IC90 values. Therefore, results demonstrated no loss of antigen binding function after PEG modification (Figure 9).

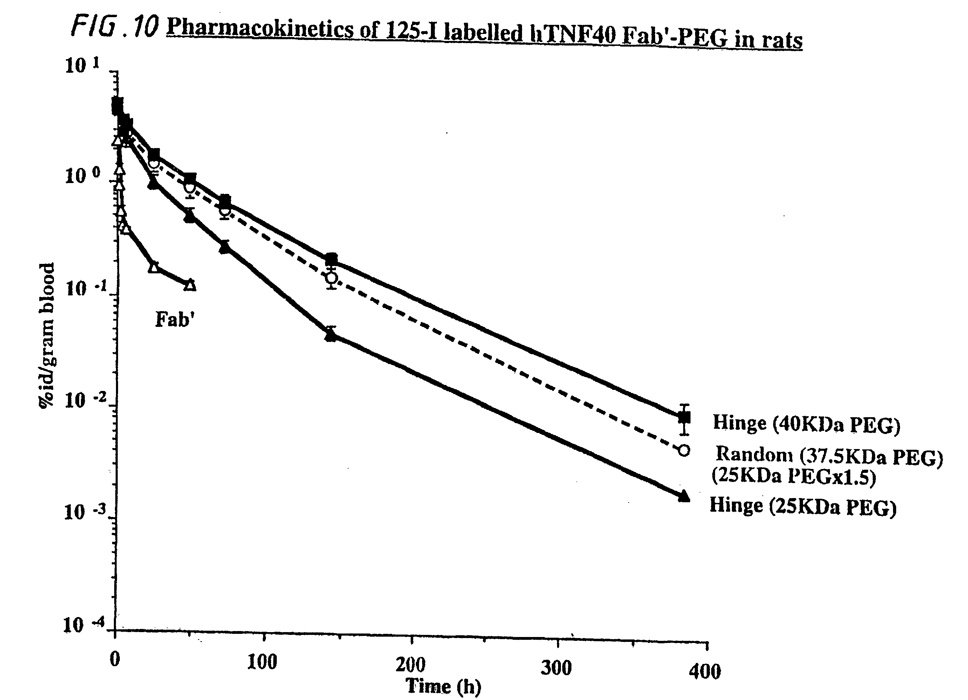

Pharmacokinetics of the purifed Fab'-PEG material and a mixture of Fab'-(PEG)₂ and Fab'-(PEG)₃ were assessed in rats after radiolabelling with ¹²⁵I. cTN3 Fab' and IgG were also compared in the same experiment. Results (Figure 10) demonstrate very rapid clearance for unmodified Fab' whereas PEG modified Fab' has a much longer circulating half-life. This is further improved by the addition of more PEG as demonstrated by the Fab'-(PEG)₂ + Fab'-(PEG)₃ sample. This sample had an increased AUC compared to IgG (Figure 10).

In Examples 5 and 6 the use of alternative thiol-selective reagents is demonstrated:

### EXAMPLE 5

### Preparation of hTNF40 Fab'-PEG using a vinyl-sulphone reagent

Vinyl-sulphones have been reported to be thiol specific when used at pH 8 or below [Morpurgo, M. et al, Bioconjugate Chem. (1996), 7, 363-368]. In this eEample, PEG is linked in a site-specific manner to Fab' using a 5kDa PEG vinyl sulphone derivative (Shearwater Polymers Inc. ibid) in which the PEG is directly attached to the sulphone group.

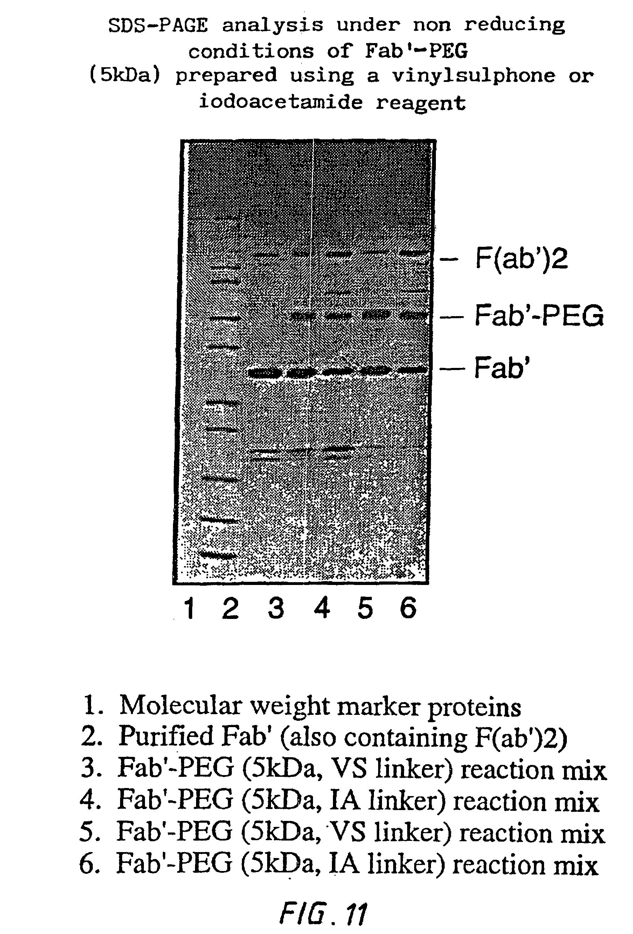

hTNF40 Fab' was prepared and reduced to generate a free hinge thiol as described in Example 2. In this preparation an average of 1.1 thiols per Fab' resulted as determined by titration with DTDP. After desalting into 0.1M phosphate buffer pH7.0 containing 2mM EDTA, a 30 fold molar excess of PEG-vinyl sulphone was added and the reaction mixture incubated overnight. SDS-PAGE analysis revealed the conjugation of PEG onto the Fab' molecule with a yield of approximately 30% (Figure 11).

Fab'-PEG was purified by hydrophobic interaction chromatography using a Phenyl-Sepharose HP Hi Trap column (Pharmacia). The cross-linking mixture was made 1.5M with respect fo ammonium sulphate and loaded onto a Phenyl-Sepharose HP column pre-equilibrated with 50mM phosphate buffer pH7.0 containing 1.5M ammonium sulphate. Fab'-PEG was eluted using a 50 column volume linear gradient to 50mM phosphate pH7. The antigen binding affinity was compared to unmodified Fab' by BIAcore analysis as described in Example 3. Results of this analysis (Table 3) demonstrated no loss of antigen binding activity through conjugation of PEG via a vinyl sulphone reagent as the binding affinity of the Fab'-PEG conjugate was similar to IgG.

### EXAMPLE 6

### Preparation of hTNF40 Fab'-PEG using an iodoacetamide reagent

In this Example, PEG is linked in a site-specific manner to Fab' using a SkDaPEG iodoacetamide derivative (Shearwater Polymers Inc. ibid) in which the PEG is directly attached to the acetamide group.

hTNF40 Fab'-PEG was prepared and purified as described in Example 5 using a 30 fold molar excess of PEG-iodoacetamide. The antigen binding affinity of the product was compared to unmodified Fab' by BIAcore analysis as described in Example 3. Results of this analysis (Table 3) demonstrated no loss of antigen binding activity through conjugation of PEG via an iodoacetamide reagent as the binding affinity of the Fab'-PEG conjugate was similar to IgG.

**Table 3.**

| **Kinetic analysis of hTNF40 Fab'-PEG conjugates** | | | |
|---|---|---|---|
| | **kass** | **kdiss** | **Kd (M)** |
| IgG standard | 4.41 x 10⁵ | 6.90 x 10⁻⁵ | 1.56 x 10⁻¹⁰ |
| Fab'-PEG (40kDA) from PEG-maleimide (Example 2) | 4.29 x 10⁵ | 7.87 x 10⁻⁵ | 1.84 x 10⁻¹⁰ |
| Fab'-PEG (5kDA) from PEG -vinyl sulphone (Example 5) | 3.69 x 10⁵ | 4.74 x 10⁻⁵ | 1.29 x 10⁻¹⁰ |
| Fab'-PEG (5kDa) from PEG-lodoacetamide (Example 6) | 3.85 x 10⁵ | 5.88 x 10⁻⁵ | 1.53 x 10⁻¹⁰ |

### EXAMPLE 7

### Preparation of anti-PDGFβR Fab'-PEG

In this example the application of site-specific PEG attachment is demonstrated with a further recombinant Fab' fragment.

Fab' from the engineered human antibody hg162, which recognises PDGFβ receptor, was expressed in E.coli as described for the Fab' fragment of hTNF40 (see Example 2). Cells were harvested from fermentation culture by centfifugation and Fab' extracted by resuspending cells in 100mM tris pH7.4 containing 10mM EDTA and incubating at 60° overnight. Fab' was then purified by expanded bed chromatography using a column of Streamline A™ (Pharmacia) which was pre-equilibrated with 1M glycine/glycinate pH8.0. The sample was made 1M with respect to glycine and the pH adjusted to 7.5 with 50% (W/v) sodium glycinate before application to the column is expanded bed mode. After washing with equilibration buffer, the column material was packed into a packed bed and Fab' was eluted with 0.1 M citrate pH3.0

Further purification was achieved by adjusting the pH of the eluate to 7.5 with 2M tris and applying to a column of Protein G sepharose pre-equilibrated with phosphate buffered saline pH7.4. After washing with equilibration buffer, Fab' was etuted with 0.1M glycine-HCI pH2.7. The pH of the eluted Fab' was then adjusted to 6.0 with 2M tris.

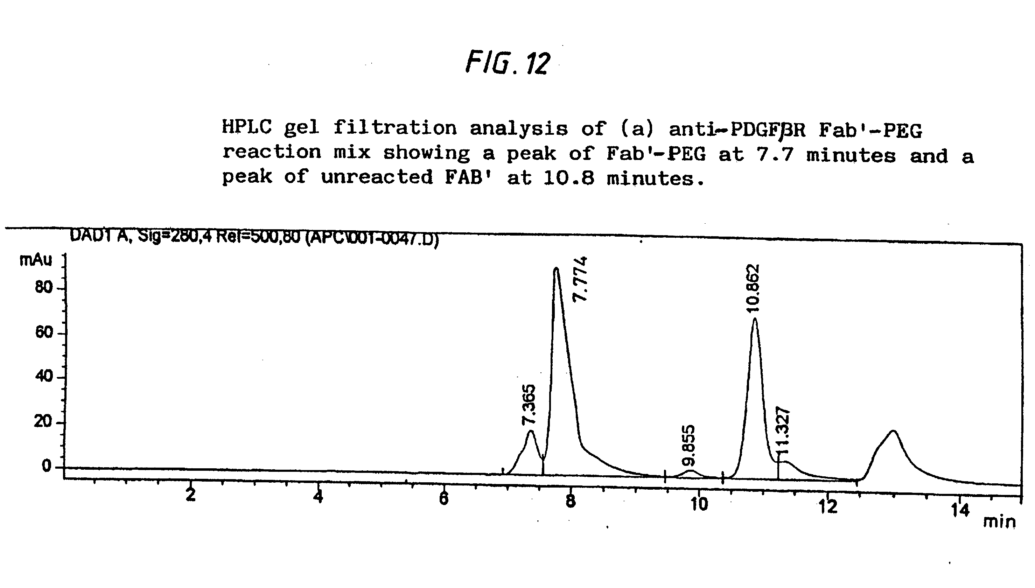

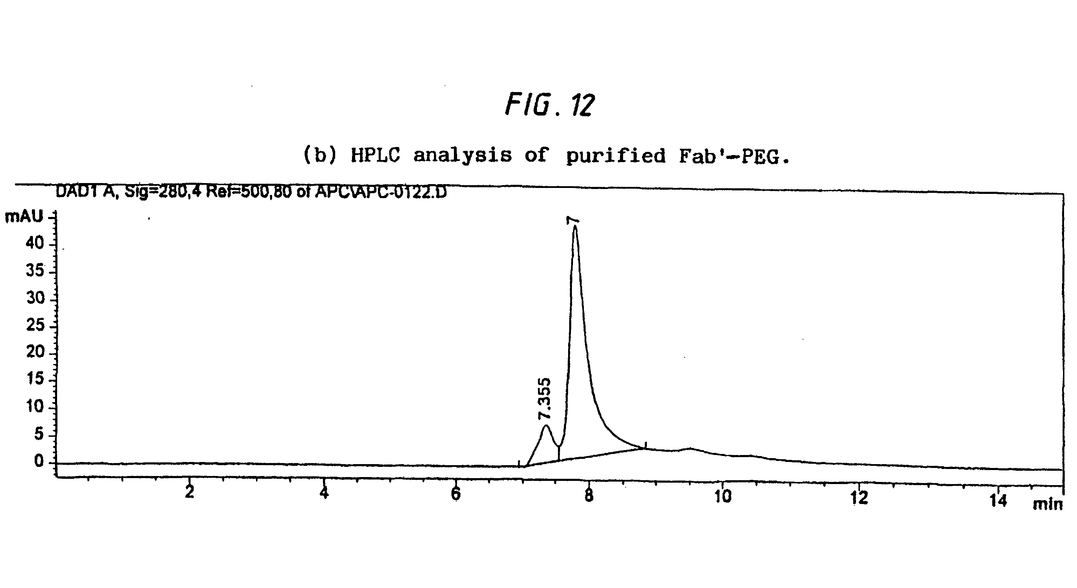

Purified anti-PDGFβR Fab' was diafiltered into 0.1M phosphate buffer, pH6.0 containing 2mM EDTA. The hinge thiol was activated by reduction with β-metcaptoethylamine. Fab' was incubated with 5mM β-mercaptoethylamine in 0.1M phosphate buffer, pH6.0 containing 2mM EDTA for 30 miunutes at 37°. The sample was then desalted into 0.1M phosphate buffer, pH6.0 containing 2mM EDTA, using Sephadex G-25 (PD10) columns. The number of thiol groups per Fab' molecule was measured by titration with DTDP, and found to be 1.08. PEG-maleimide (40kDa See Example 2) was then added at a three fold molar excess and allowed to react overnight. Conversion to Fab'-PEG was achieved with a yield of approximately 60% (Figure 12). Fab'-PEG conjugate was then purified by gel filtration HPLC as described in Example 2.

Antigen binding affinity of anti-PDGFβR Fab'-PEG was compared to unmodified Fab' by BlAcore analysis. Kinetic analysis to determine the on and off rates for anti-PDGFβR Fab'-PEG binding to PDGFβR was performed using a BIACORE 2000 (Biacore AB). A mouse IgG Fc-PDGFβR fusion molecule was captured by an anti-mouse IgG immobilised on the sensor chip surface. This was followed by injection of anti-PDGFβR Fab'-PEG. Affinipure F(ab')₂ fragment of goat anti-mouse Ig, Fc fragment specific (Jackson ImmunoResearch) was immbolised on a Sensor Chip CM5 via amine coupling chemistry to a level of 11500RU. A blank surface was prepared by following the immbolisation procedure but omitting injection of the capturing molecule. HBS buffer (10mM HEPES pH7.4, 0.15M NaCl, 3mM EDTA, 0.005% Surfactant P20, Biacore AB) was used as the running buffer with a flow rate of 10µl/min. An injection of mouse IgG Fc-PDGFβR expressed in recombinant COS cell supematant was captured by the immobilised anti-mouse IgG to a level between 200-250RU. Anti-PDGFβR Fab' or Fab'-PEG molecules were titrated over the captured mouse IgG Fc-PDGFβR surface from 2mg/ml to 0.52mg/ml. Surfaces were regenerated by injecting 10ml of 30mM hydrochloric acid. Injections of mouse IgG Fc-PDGFβR and each concentration of anti-PDGFβR Fab' or Fab'-PEG were repeated over the blank surface as controls. The sensorgram for each anti-PDGFβR Fab' or Pab'-PEG concentration was corrected with the corresponding sensorgram for the blank surface after deleton of the mouse IgG Fc-PDGFβR injection and regeneration step. Kinetic parameters were calculated using BlAevaluation 2.1 software.

Results for Fab' and Fab'-PEG are shown in Table 4. There was little difference in the values of the kinetic parameters determined, demonstrating that attachment of PEG at the hinge region has resulted in little loss of antigen binding affinity.

**Table 4**

| **BlAcore analysis of anti-PDGFβR Fab' and Fab'-PEG** | | | |
|---|---|---|---|
| | **kass** | **kdiss** | **Kd (M)** |
| Fab' | 6.89 x 10⁶ | 2.52 x 10⁻³ | 3.66 x 10⁻¹⁰ |
| Fab'-PEG | 4.45 x 10⁶ | 2.77 x 10⁻³ | 6.22 x 10⁻¹⁰ |

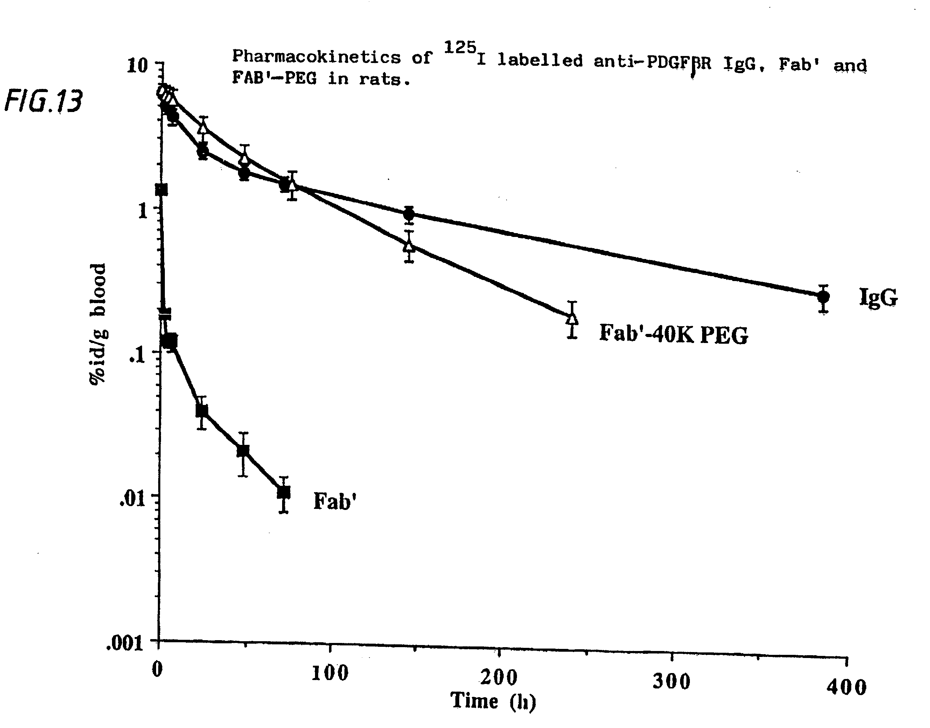

Pharmacokinetics of anti-PDGFβR Fab' and Fab'-PEG were examined in a rat experiment using ¹²⁵I-labelled samples as described in Example 1. Results demonstrated much slower clearance from the blood for Fab'-PEG compared to Fab' (Figure 13). This was reflected in the calculations of pharmacokinetic parameters shown in Table 5.

**Table 5**

| **Pharmacokinetic parameters of anti-PDGFβR Fab'-POEG compared to Fab' and IgG.** | | | | |
|---|---|---|---|---|
| | **t1/2 α (hours)** | **t1/2 β (hours)** | **AUC(0-) (%dose x h)** | **AUC (% of IgG value)** |
| **IgG** | 5.3 +/- 1.3 | 95.9 +/- 10.9 | 6442 +/- 525 | 100 |
| **Fab'** | 0.35 +/- 0.01 | 20.3 +/- 6.0 | 90 +/- 12 | 1.4 |
| **Fab'-PEG (40kDa)** | 8.9 +/- 4.7 | 49.1 +/- 4.8 | 5890+/-1296 | 91 |

## Claims

1. A modified monovalent antibody fragment comprising a heavy chain and a light chain, wherein:
said heavy chain consists of a V_{H} domain covalently linked at its C-terminus to a C_{H}1 domain;
said light chain consists of a V_{L} domain, which is complementary to the V_{H} domain, covalently linked at its C-terminus to a C_{L} domain;
said CH1 domain is extended to provide a hinge domain which contains only one cysteine residue;
the cysteine residues in the V_{H}, C_{H}1, V_{L} and C_{L} domains are in disulphide linkage to each other; and
the cysteine residue in the hinge domain is covalently linked through its sulphur atom to a polymer molecule.

2. An antibody fragment according to claim 1 wherein the polymer is an optionally substituted straight or branched chain polyalkylene, polyalkenylene or polyoxyalkylene polymer or a branched or unbranched polysaccharide.

3. An antibody fragment according to claim 2 wherein the polymer is an optionally substituted straight or branched chain poly(ethylene glycol), polypropylene glycol) or poly(vinyl alcohol) or a derivative thereof.

4. An antibody fragment according to claim 3 wherein the polymer is methoxy(polyethylene glycol) or a derivative thereof.

5. An antibody fragment according to any one of claims 1 to 4 covalently attached to one or more effector or reporter molecules.

6. A pharmaceutical composition comprising a monovalent antibody fragment according to any one of the preceding claims together with one or more pharmaceutically acceptable excipients, diluents or carriers.

## Patentansprüche

1. Modifiziertes, monovalentes Antikörperfragment, welches eine schwere Kette und eine leichte Kette umfaßt, wobei
die schwere Kette aus einer über ihren C-Terminus mit einer C_{H}1-Domäne kovalent verknüpften V_{H}-Domäne besteht,
die leichte Kette aus einer V_{L}-Domäne besteht, welche komplementär zu der über ihren C-Terminus mit einer C_{L}-Domäne kovalent verknüpften VH-Domäne ist,
die C_{H}1-Domäne zur Bereitstellung einer Gelenkdomäne, welche nur einen Cysteinrest enthält, verlängert ist,
die Cysteinreste in den V_{H}-, C_{H}1-, V_{L}- und C_{L}-Domänen Disulfid-Bindungen miteinander aufweisen und
der Cysteinrest in der Gelenkdomäne über sein Schwefelatom mit einem Polymermolekül kovalent verknüpft ist.

2. Antikörperfragment nach Anspruch 1, wobei das Polymer ein gegebenenfalls substituiertes, geradkettiges oder verzweigtkettiges Polyalkylen-, Polyalkenylen- oder Polyoxyalkylenpolymer oder ein verzweigtes oder unverzweigtes Polysaccharid ist.

3. Antikörperfragement nach Anspruch 2, wobei das Polymer ein gegebenenfalls substituiertes, geradkettiges oder verzweigtkettiges Poly(ethylenglykol), Poly(propylenglykol) oder Poly(vinylalkohol) oder ein Derivat davon ist.

4. Antikörperfragment nach Anspruch 3, wobei das Polymer Methoxy(polyethylenglykol) oder ein Derivat davon ist.

5. Antikörperfragment nach einem der Ansprüche 1 bis 4, welches an ein oder mehrere Effektor- oder Reportermoleküle kovalent gebunden ist.

6. Pharmazeutische Zusammensetzung, welche ein monovalentes Antikörperfragment nach einem der vorhergehenden Ansprüche zusammen mit einem oder mehreren pharmazeutisch verträglichen Arzneimittelträgern, Verdünnungsmitteln oder Trägern umfaßt.

## Revendications

1. Fragment d'anticorps monovalent modifié comprenant une chaîne lourde et une chaîne légère, dans lequel:
ladite chaîne lourde est constituée d'un domaine V_{H} lié de manière covalente à son extrémité C-terminale à un domaine C_{H}1;
ladite chaîne légère est constituée d'un domaine V_{L} qui est complémentaire au domaine V_{H} et lié de manière covalente à son extrémité C-terminale à un domaine C_{L};
ledit domaine C_{H}1 est étendu pour procurer un domaine charnière qui ne contient qu'un seul résidu cystéine;
les résidus cystéine des domaines V_{H}, C_{H}1, V_{L} et C_{L} sont liés les uns aux autres en formant une liaison disulfure; et
le résidu cystéine du domaine charnière est lié de manière covalente par l'intermédiaire de son atome de soufre à une molécule polymère.

2. Fragment d'anticorps selon la revendication 1, dans lequel le polymère est un polymère polyalkylène, polyalcénylène ou polyoxyalkylène à chaîne linéaire ou ramifiée, éventuellement substitué, ou un polysaccharide ramifié ou non ramifié.

3. Fragment d'anticorps selon la revendication 2, dans lequel le polymère est un poly(éthylèneglycol), poly(propylèneglycol) ou poly(alcool vinylique) à chaîne linéaire ou ramifiée, éventuellement substitué, ou un de leurs dérivés.

4. Fragment d'anticorps selon la revendication 3, dans lequel le polymère est un méthoxy(polyéthylèneglycol) ou un dérivé de celui-ci.

5. Fragment d'anticorps selon l'une quelconque des revendications 1 à 4, lié de manière covalente à une ou plusieurs molécules effectrices ou indicatrices.

6. Composition pharmaceutique comprenant un fragment d'anticorps monovalent selon l'une quelconque des revendications précédentes, ainsi qu'un ou plusieurs excipients, diluants ou véhicules acceptables sur le plan pharmaceutique.
